# EUROPEAN PATENT APPLICATION

(11) **EP 3 413 262 A1**
(43) Date of publication of application: **12.12.2018**
(21) Application number: 15910276.3
(22) Date of filing: 11.12.2015
(51) Int. Cl.: G06Q 50/00, G01N 30/86

(54) **ANALYSIS INFORMATION MANAGEMENT SYSTEM**

(71) Applicant: Shimadzu Corporation, Kyoto-shi, Kyoto 604-8511 (JP)
(72) Inventor: ONO, Koji, Kyoto-shi Kyoto 604-8511 (JP)
(74) Representative: Kilian Kilian & Partner
(86) International application number: PCT/JP2015/084831
(87) International publication number: WO 2017/098661

(57) **Abstract**

In the case where a plurality of analysis data files which respectively contain analysis results and data-analyzing process results for a plurality of samples should be managed as one lot, a portion of the information contained in those analysis data files is extracted and compiled into a final result record report file. This file is registered in a database. A supervisor views the final result record report on a client terminal, evaluates the validity of the analysis result and other contents for each sample, and performs a predetermined operation to execute the signature of approval or other evaluations. Signature information, including the signer's name, date and time of the signature, etc., is recorded for the final result record report file. The same signature information is also recorded for each of the analysis data files located by referring to link information. The supervisor does not need to execute the signature for each individual analysis data file contained in one lot. This helps improve the efficiency of the task of data-checking and signing.

## Description

### TECHNICAL FIELD

The present invention relates to an analysis information management system for storing and managing data collected with various analytical instruments, such as a chromatograph apparatus, mass spectrometer or spectrophotometer, as well as calculated values and other process results obtained by a data-analyzing process performed on those data.

### BACKGROUND ART

In recent years, most of the tasks of processing and managing various data acquired with an analytical instrument, such as a gas chromatograph (GC), liquid chromatograph (LC) or mass spectrometer, have been performed by using a computer on which dedicated software was installed. In particular, a large-scale system in which multiple analytical instruments and computers (e.g. client terminals) as well as a database server and other devices are connected to each other through a communication network has also been proposed in recent years to meet various needs, such as the sophistication of analysis, automatic and efficient analyzing tasks, as well as the necessity for a centralized management of the measuring task and data-analyzing task. Typically, in such an analysis information management system, the data obtained for a sample in an analytical instrument, or process results obtained by analytically processing the data on a client terminal or the like, such as calculated values (e.g. quantitative values), are stored in a single data file for each sample. The data file is registered in, and managed on, a database constructed on a database server or similar location (see Non-Patent Literature 1 and other documents).

Such an analysis information management system has the function of automatically creating an analysis report in which analysis data obtained by analyzing a sample with an analytical instrument, a graph or table created based on those data, as well as various calculated values (e.g. quantitative values) determined by analytically processing those data, are organized in a predetermined form. This report can be provided in the form of a hard copy as well as registered in a database as an electronic file in PDF format or the like (see Non-Patent Literature 2 or other documents).

In many cases, the task of preparing an analysis report in the previously described manner is performed by an operator who took charge of the measuring task. It is often the case that the operator merely performs necessary tasks following a routine described in a manual or the like. Therefore, an analysis report prepared by an operator may have some problems, such as an incorrectly inserted graph or the like, or an omission of some item of information which needs to be reported. Accordingly, in many cases, an analysis report which has been prepared by an operator according to a predetermined procedure is subsequently subjected to a checking process in which another individual with a higher level of expertise (who is hereinafter called the "checker") checks the contents of the analysis report and determines whether or not there is any problem with its content. If a problem has been found in an item of information, the checker informs the operator of the problem, who reconstruct the analysis report to complete the final version of the analysis report which is free of any problems.

After the analysis report has been completed, either the checker or an individual having an even higher level of administrative authority than the checker (who is hereinafter called the "supervisor") normally checks the data including the analysis report visually, and gives his/her signature which indicates that the signer has approved (or cannot approve) the report. For such a task, a conventional analysis information management system has the function of electronic signature, which allows for electronic recording of the signer's name, date and time of the signature, reason for the signature as well as other relevant information for an analysis data file or the like stored in a database (see Non-Patent Literature 1).

In recent years, in an LC analysis or GC analysis, an analytical instrument has often been used which continuously and automatically performs an analysis on each sample while replacing one sample with another using an autosampler in which many samples can be loaded beforehand. In an analysis using such an instrument, it is often the case that a plurality of samples are collectively evaluated from the result of a data-analyzing process based on a plurality of sets of analysis data respectively acquired for those samples by a continuous analysis. For example, in the case of investigating a temporal change in the concentrations of a plurality of compounds in a solution, a number of samples are prepared by collecting the solution in small quantity at predetermined intervals of time. An LC analysis is performed on each of those samples to determine the quantity of each target compound, and the temporal change in the concentration of the target compound is evaluated from the quantitative values obtained for that compound.

For such a test, managing the analysis data and the data-analyzing process results obtained for one sample is insufficient; it is essential that the analysis data and data-analyzing process results obtained for a number of samples in one continuous analysis be managed in a collective form, i.e. as one lot. However, as explained earlier, the analysis data file is normally created for each individual sample. Therefore, in the case where analysis data files respectively obtained for a plurality of samples are managed as one lot, the supervisor needs to check the content of each of the analysis data files included in the lot on a display screen or the like, and give his/her signature. If there are a large number of analysis data files in one lot, such a task related to the signature will be extremely burdensome and possibly cause an incorrect check of data, omission of the check, omission of the signature, or other problems. The task of checking whether or not all analysis data files included in one lot have been approved will also be cumbersome and time-consuming, since those analysis data files need to be individually checked for the signature.

### CITATION LIST

### NON PATENT LITERATURE

Non-Patent Literature 1: "CLASS-Agent Ver. 2 Nettowaaku Taiou Bunseki Deeta Kanri Tsuuru (Class-Agent Ver. 2, Network-Compatible Analysis Data Management Tool)", [online], Shimadzu Corporation, [accessed on December 7, 2017], the Internet <URL: http://www.an.shimadzu.cojp/data-net/class-agent_ver2/index.htm>

Non-Patent Literature 2: "LabSolutions Sougou: Kinou Shoukai Repooto Sakusei (LabSolutions General: Introduction to Functions - Report Preparation)", [online], Shimadzu Corporation, [accessed on December 7, 2015], the Internet <URL: http://www.an.shimadzu.co.jp/data-net/labsolutions/function/7_smart_validated-report.htm>

### SUMMARY OF INVENTION

### TECHNICAL PROBLEM

The present invention has been developed to solve the previously described problem. Its objective is to provide an analysis information management system with which users can easily perform the task of electronic signature for an analysis report or similar electronic files related to an analysis, as well as efficiently check the result of the signature, in the case where analysis results and data-analyzing process results for a plurality of samples need to be collectively managed.

### SOLUTION TO PROBLEM

The present invention developed for solving the previously described problem is an analysis information management system including a communication network, an analytical instrument connected to the communication network, a database server connected to the communication network, as well as a client terminal connected to the communication network and provided with an operation unit and a display unit, the analysis information management system configured to register, in a database constructed on the database server, a data file containing analysis data acquired by performing an analysis on a sample with the analytical instrument and/or data-analyzing process result acquired by performing a data-analyzing process based on the analysis data using the client terminal, and to manage the registered data file, in which:
the client terminal includes:
a) an integrated file creator for extracting at least a portion of a plurality of pieces of information respectively contained in a plurality of data files registered in the database or a plurality of pieces of information respectively associated with the data files, for compiling the extracted pieces of information into one integrated file, and for storing the integrated file in the database;
b) a signature-receiving processor for reading the integrated file from the database and displaying the pieces of information contained in the integrated file on the display unit, and for receiving a predetermined operation with the operation unit by a user viewing the displayed information, as a command to execute a signature for the integrated file; and
c) a signature-executing processor for performing a recording of the signature for the integrated file and for performing a recording of the signature for each of the data files from which the integrated file is created, when the command to execute the signature is received by the signature-receiving processor.

In the analysis information management system according to the present invention, the system may include a plurality of analytical instruments and a plurality of client terminals, and the database server and one of the client terminals (or the sole client terminal when the system includes a single client terminal) may be located on a single hardware unit. In other words, a computer serving as the client terminal may be configured to additionally function as the database server. The analytical instrument (or instruments) may be any type of device as long as it can perform an analysis and acquire a certain kind of data.

In the analysis information management system according to the present invention, for example, when a plurality of data files which need to be evaluated as one set of analysis results are selected by a user, the integrated file creator reads the selected data files from the database and extracts predetermined pieces of information respectively contained in or associated with those data files. The user may preferably be allowed to previously specify the pieces of information to be extracted. It is desirable that those pieces of information be such kinds of information based on which users can evaluate or check the validity of the substantial contents of the data file, such as the data obtained by an analysis or results of a data-analyzing process. For example, if the analytical instrument is an LC, those pieces of information should preferably include a chromatogram, a peak table showing peak information obtained by the waveform processing of the chromatogram, and the result of quantitative determination calculations.

Subsequently, the integrated file creator compiles the pieces of information extracted from the data files into one integrated file and registers it in the database. This integrated file may preferably be created as an electronic file in PDF (portable document format) or similar format in which a document containing graphs, tables and other elements can be visually presented in an almost the same appearance as a printed version. Needless to say, the integrated file may contain any kind of information other than the substantial contents of the data files. For example, an audit trail, which contains a collection of log information related to the data files, may be included in the integrated file.

For example, when a supervisor having a high level of administrative authority needs to check or evaluate the contents of the integrated file, the supervisor selects and indicates the integrated file, whereupon the signature-receiving processor reads the indicated integrated file from the database and displays the information contained in the file on the screen of the display unit. Viewing the screen, the supervisor checks the information extracted from each of the original data files and performs a predetermined operation with the operation unit to give his/her signature which, for example, indicates that the content of the integrated file has been approved. The signature-receiving processor receives this operation as a command to execute the signature for the integrated file. In response to this, the signature-executing processor performs the recording of the signature for the integrated file as well as for each of the data files from which the integrated file has been created.

The "recording of the signature" specifically means the process of registering, in the database, a new file created by adding the signer's name, date and time of the signature, reason for the signature (approval or non-approval) and other relevant information to the original file (e.g. integrated file, data file or other files), or the process of updating a file which holds a collection of signature information corresponding to the integrated files and data files. In any case, the signature information is associated with the original files and managed on the database.

In this manner, with the analysis information management system according to the present invention, the supervisor only needs to view the information contained in one integrated file and perform an operation for the signature of approval, non-approval or the like for the integrated file. Upon this operation, the signature of approval, non-approval or the like is automatically completed for not only the integrated file but also all data files from which the integrated file has been created.

In the analysis information management system according to the present invention, the integrated file creator may preferably be configured to create link information showing the correspondence relationship between the integrated file and the data files from which the integrated file is created, and to store the link information in the database.

In the above-described configuration, the signature-receiving processor may be configured to prepare a list of information for identifying the data files corresponding to the integrated file or information related to the data files, with reference to the link information, and display the list on the display unit.

With this configuration, when a user selects an intended integrated file from the integrated files registered in the database, the system can provide the user with information concerning the data files corresponding to the integrated file which is about to be selected. This helps the user easily select the correct integrated file.

### ADVANTAGEOUS EFFECTS OF INVENTION

With the analysis information management system according to the present invention, the user only needs to perform the content-checking and signing operations for one integrated file which reflects analysis results or data-analyzing process results obtained for a plurality of samples contained in one lot which should be collectively handled. In response to those user operations, the system automatically executes the signature for the individual data files which respectively contain the analysis results or data-analyzing process results for the plurality of samples. Unlike the case of the conventional system, it is unnecessary to perform the content-checking and signing operations for each individual data file. Accordingly, the tasks related to the approval of data and the signing can be efficiently performed. Incorrect operations or signature omissions which possibly occur in those tasks will also be decreased.

The signature information for an integrate file is identical with the signature information for the data files corresponding to that integrated file. Therefore, by checking the signature information for the integrated file, the user can check, for example, whether or not the entire lot has been approved. Unlike the case of the conventional system, it is unnecessary to check signature information for each of the data files which are managed as one lot. Thus, the efficiency of the signature-checking process will also be improved.

### BRIEF DESCRIPTION OF DRAWINGS

Fig. 1 is a schematic configuration diagram of one embodiment of the analysis information management system according to the present invention.
Fig. 2 is a flowchart showing the final result record creation process in the analysis information management system according to the present embodiment.
Fig. 3 is a model diagram showing one example of the form of a final result record report.
Fig. 4 is a model diagram showing one example of a data manager window for final result record creation.
Fig. 5 is a model diagram showing another example of the data manager window for final result record creation.
Fig. 6 is a conceptual diagram showing the relationship of files in the case of handling a plurality of analysis data files as one lot.
Fig. 7 is a diagram illustrating an operation of a signature-executing process in the case of handling a plurality of analysis data files as one lot.
Fig. 8 is a diagram showing one example of a display window for viewing the link between a plurality of analysis data files and a final result record report file.
Fig. 9 is a diagram showing one example of an analysis report.
Fig. 10 is a diagram showing one example of an audit trail report.
Fig. 11 is a diagram showing one example of a data list.

### DESCRIPTION OF EMBODIMENTS

One embodiment of the analysis information management system according to the present invention is hereinafter described with reference to the attached drawings.

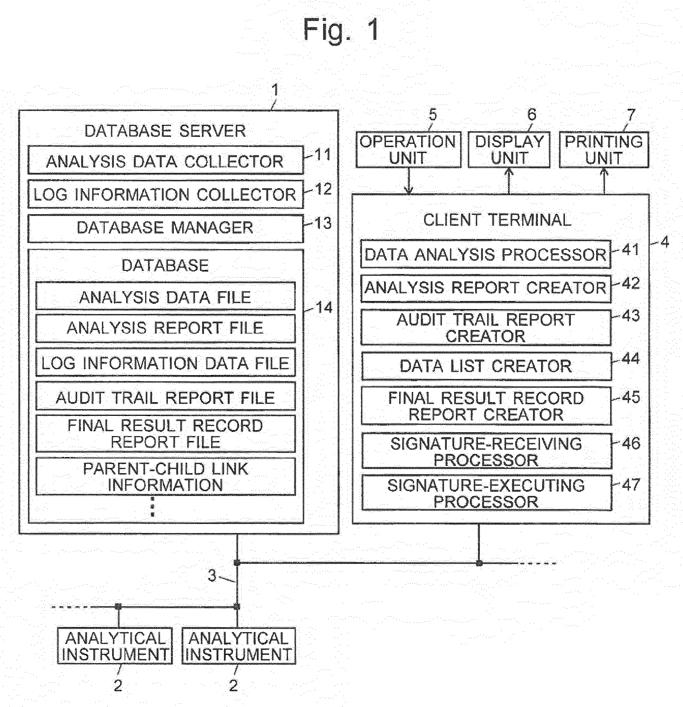

Fig. 1 is a schematic configuration diagram of the main components of the analysis information management system according to the present embodiment.

The analysis information management system according to the present embodiment includes: one or more analytical instruments 2 for performing a predetermined analysis on a sample and collecting measurement data; a database server 1 which is actually a computer; and a client terminal 4 which is actually a personal computer. These three kinds of devices are connected to each other through a communication network 3, such as a local area network (LAN). An operation unit 5, which includes a mouse, keyboard and other devices, as well as a display unit 6 and a printing unit 7 are connected to the client terminal 4. Normally, an operation unit, display unit and other devices are also connected to the database server 1, although those units are not shown in Fig. 1.

Although the configuration shown in Fig. 1 has a single client terminal 4, there may be multiple client terminals. The client terminal 4 may additionally function as the database server 1. Each analytical instrument 2 may have built-in functions which effectively work similar to a computer and enable direct connection to the communication network 3. However, more typically, the analytical instrument is connected to the communication network 3 via a personal computer (not shown). In that case, two or more analytical instruments 2 may be connected to a single personal computer which is connected to the communication network 3. Such a personal computer may be configured to serve as a client terminal 4. The type of analytical instruments 2 is not specifically limited. Different types of analytical instruments may be connected to the communication network 3. For convenience of description, it is hereinafter assumed that the analytical instruments 2 are all liquid chromatograph (LC) apparatuses.

The database server 1 includes an analysis data collector 11, log information collector 12, database manager 13, database 14 and other functional blocks. The database 14 is used to register various kinds of files and information, such as the analysis data file, analysis report file, log information data file, audit trail report file, final result record report file, and parent-child file link information. The client terminal 4 includes a data analysis processor 41, analysis report creator 42, audit trail report creator 43, data list creator 44, final result record report creator 45, signature-receiving processor 46, signature-executing processor 47 and other functional blocks. As noted earlier, the database server 1 and the client terminal 4 are actually computers. Therefore, the aforementioned functional blocks are embodied by dedicated programs which are previously installed on those computers and executed on the same computers.

In the database server 1, the analysis data collector 11 collects data acquired in each analytical instrument 2 by an LC analysis on a sample as well as various items of information related to the analysis through the communication network 3. The collected data and information are registered through the database manager 13 in the database 14 as an analysis data file. When the analysis data file is registered, a data ID for uniquely identifying the file is automatically given to the file. The analysis data file normally contains various items of data and information related to an analysis on one sample. That is to say, in addition to the analysis data acquired through an analysis, those items of data and information include: sample information, such as the name and amount of the sample; device ID for identifying an analytical instrument used for the analysis; date and time of the analysis; analyzer user ID for identifying an operator who carried out the analysis; analysis conditions applied in the analysis (e.g. the flow velocity of the mobile phase); and calibration curve information for quantitative determination. The same analysis data file may additionally be used to store various kinds of calculated values obtained through a data-analyzing process based on the analysis data by the data-analyzing processor 41 in the client terminal 4 as well as other related items of information.

The log information collector 12 collects various items of information through the communication network 3 and registers the information through the database manager 13 in the database 14 as the log information data file. The items of information include operations and tasks performed by users on all analytical instruments 2, client terminal 4 and other devices included in the present system, as well as information indicating the state of each device, such as an error which occurred in an analytical instrument 2, client terminal 4 or other devices during an execution of a measurement or data-analyzing process. For example, the operation log data file, which shows operation logs, contains various kinds of data and information related to each specific operation: content of the operation; device ID for identifying an analytical instrument 2 or client terminal 4 on which the operation was performed; date and time of the operation; and user ID for identifying an operator who performed the operation.

Next, various processing operations to be performed on the client terminal 4, with analysis data files stored in the database 14, are described.

### [Analysis Report Creation Process]

The analysis report creator 42 in the client terminal 4 automatically creates an analysis report in a predetermined form based on the various data and information contained in an analysis data file, and registers the analysis report in the database 14 as an analysis report file in PDF format.

Specifically, an operator in charge of an analysis operation performs a predetermined operation with the operation unit 5 to select an analysis data file for which an analysis report should be created and a template for the same report, and issues a command to create the report. Then, the analysis report creator 42 begins to operate and accesses the database server 1 to read the report template concerned and the specified analysis data file from the database 14. A report template is a form (format) of a report in which various report items, such as the sample information, chromatogram and peak report, are appropriately arranged. The provider (manufacturer) of the present system can previously create such templates and register them in the database 14.

The analysis report creator 42 creates an analysis report by inserting necessary information into the information presentation field of each report item in the read report template. Examples of the inserted information include; a chromatogram created from the data contained in the analysis data file; a peak table showing the information concerning the peaks on the chromatogram obtained by performing a data-analyzing process on the chromatogram; and sample information contained in the analysis data file. The analysis report may also include a check result entry field for each report item to allow a checker, who is different from the operator, to check the content of the analysis report and enter the check result as well as describe the reason for the rejection of the content if the checker has concluded that the reported content is unacceptable.

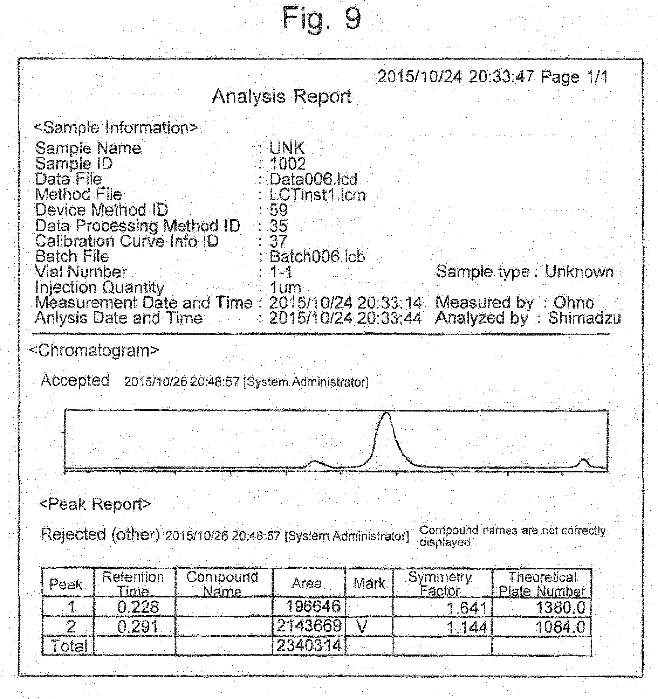

Fig. 9 is a diagram showing one example of the analysis report which is automatically created. In the present example, there are three report items, i.e. the sample information, chromatogram and peak report, in which the chromatogram and peak report are each provided with a check result entry field.

A method for creating a report template in which check result entry fields are provided is described, for example, in the Japanese Patent Application No. 2015-239016, which is a prior application filed by the applicant.

### [Audit Trail Report Creation Process]

The audit trail report creator 43 in the client terminal 4 automatically collects various pieces of log information related to one or more analysis data files, creates a list showing those pieces of log information arranged in time-series order, and registers an audit trail report inclusive of the list in the database 14 as an audit trail report file in PDF format.

Specifically, an operator performs a predetermined operation with the operation unit 5 to select analysis data files for which an audit trail should be created, and issues a command to create the audit trail. Then, the audit trail report creator 43 begins to operate and accesses the database server 1 to read all log information files related to the specified analysis data files. The "all log information files" contain two types of log information: the log information identified by the device ID of the analytical instrument with which the data contained in the analysis data files were acquired and the user ID of an analysis operator who took charge of the analysis, obtained within a period of time from login to logout including the date and time of the creation or registration of the analysis data files; and the log information which indicates a certain kind of file operation performed on each specified analysis data file.

The former type of log information can be collected by searching the log information in the database 14 using, as search keys, the device ID, user ID as well as date and time of creation (or registration) contained in the analysis data files or associated with those files as property information for example. The latter type of log information can be collected by searching the log information in the database 14 using, as a search key, the data ID associated with each analysis data file. It is also possible to issue a login ID which allows for unique identification of a time range from a login operation to the logout on any of the devices in the present system, i.e. the analytical instruments 2, client terminal 4 and other devices, and to add the login ID to every log information data file and every analysis data file. This facilitates the extraction of the log information related to an analysis data file.

The audit trail report creator 43 creates an audit trail by organizing a large number of pieces of log information extracted in the previously described manner in time-series order, and creating a list which shows messages that briefly describe the contents of the respective pieces of log information along with a portion of the log information.

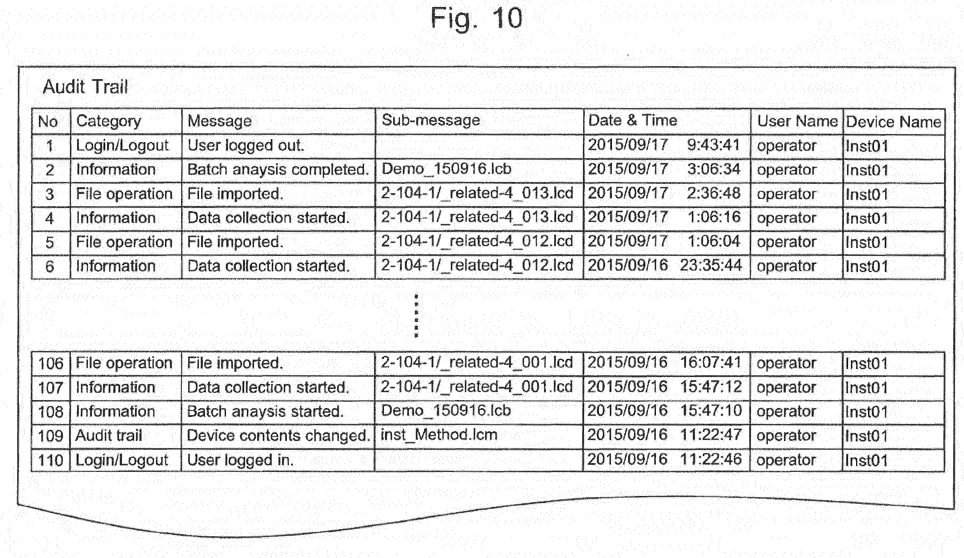

Fig. 10 is a diagram showing one example of the audit trail report which is automatically created.

### [Final Result Record Report Creation Process]

Next, a processing operation for creating a final result record report is described. This report includes the information of the analysis report based on the data contained in an analysis data file and the information of an audit trail report. Although the final result record report may be created for a single sample, the following description deals with the case of creating a final result record report which reflects analysis results, data-analyzing process results and other data obtained for a plurality of samples by a continuous analysis. Such a final result record report is useful in the case where a plurality of samples which are handled as one lot should be collectively evaluated based on analysis results and data-analysis results obtained for those samples.

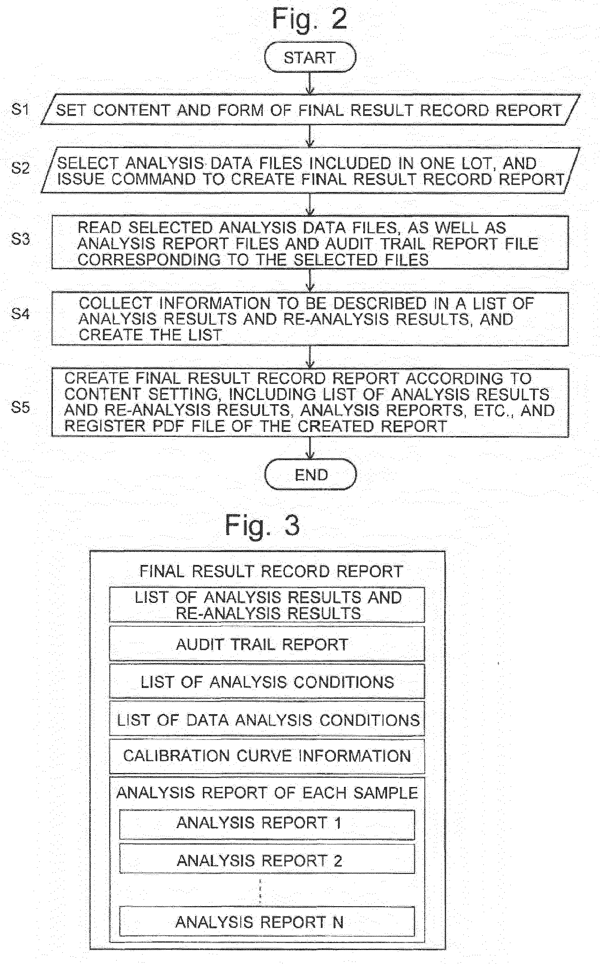

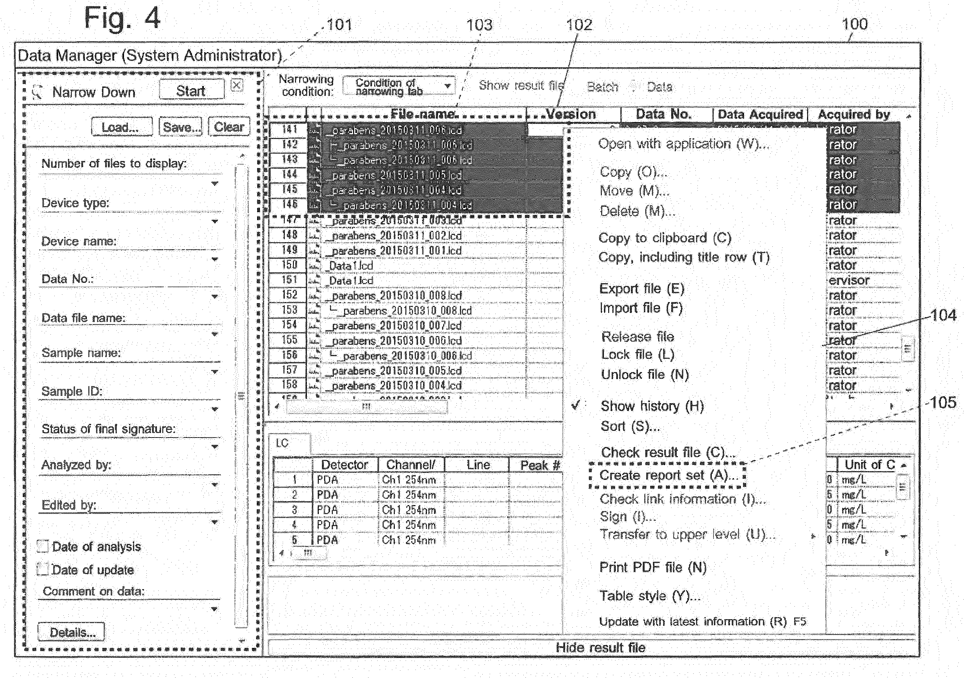

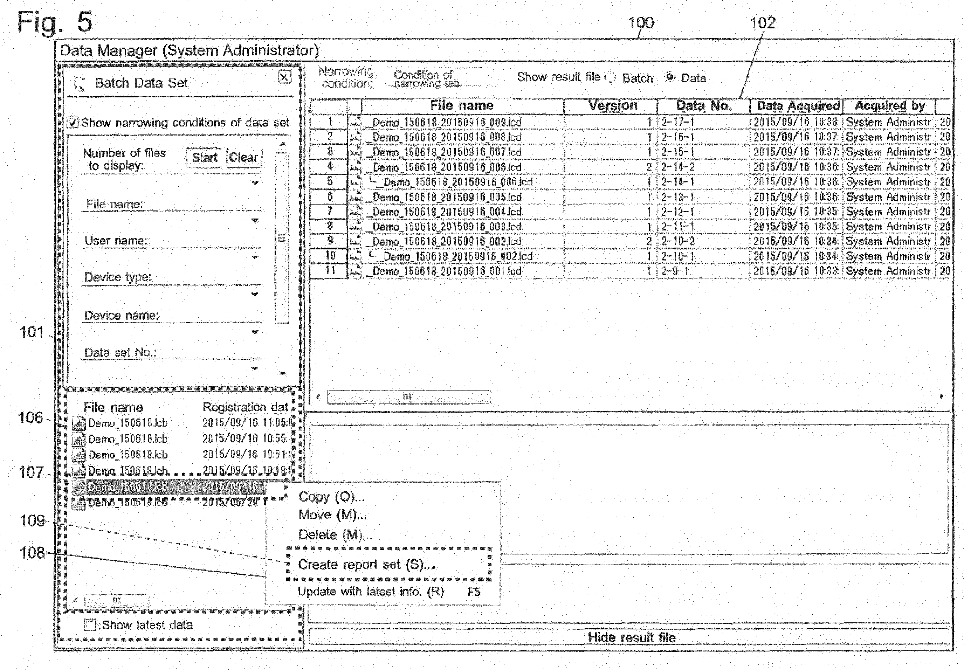

Fig. 2 is a flowchart showing the final result record creation process. Fig. 3 is a model diagram showing one example of the form of a final result record report. Fig. 4 is a model diagram showing one example of a data manager window for final result record creation. Fig. 5 is a model diagram showing another example of the data manager window for final result record creation.

In advance of the creation of the final result record, an operator performs a predetermined operation with the operation unit 5 of the client terminal 4 to specify the contents and form of the final result record report (Step S1). For example, in the case of Fig. 3, the final result record report can contain various items of information described in the following order: a list of analysis results and re-analysis results, audit trail report, analysis condition list, data-analysis condition list, calibration curve information, as well as analysis reports for individual samples. The items and form of the report may be appropriately changed; for example, the order of the items may be appropriately changed, some of those items may be removed, or new items may be added. Step S1 may be bypassed if the default setting of the contents and form of the final result record report can be used as it is, or if there is no need to change the previously used setting.

Subsequently, the operator performs a predetermined operation with the operation unit 5 of the client terminal 4, whereupon the final result record report creator 45 begins to operate and displays a data manager window 100 for final result record creation on the screen of the display unit 6. As shown in Fig. 4, the data manager window 100 has a file selection setting area 101 located in its left part and a data file list 102 located in its upper right part. The setting in the file selection setting area 101 can be selected from two modes: "narrow down" and "batch data set". Fig. 4 is the case of the "narrow down" setting, while Fig. 5 is the case of the "batch data set" setting.

In the case of using the "narrow down" setting in the file selection setting area 101 as shown in Fig. 4, the operator sets the narrowing conditions in one or more narrowing items in the file selection setting area 101. Then, the final result record report creator 45 accesses the database 14 to search for analysis data files which satisfy the narrowing conditions, and displays a list of the thereby found analysis data files in the data file list 102. It is naturally possible to refine the search with additional narrowing conditions and decrease the number of analysis data files if there is an excessive number of analysis data files displayed in the data file list 102.

The operator specifies a plurality of analysis data files included in one lot for which the final result record report should be created among the analysis data files displayed in the data file list 102, by a clicking operation with a mouse on the screen. In Fig. 4, the six analysis data files denoted by reference sign 103 are selected. The selected files are highlighted. Then, for example, the operator presses the right button of the mouse on the selected items, whereby an operation menu 104 as shown in Fig. 4 is displayed. The operator selects the "Create report set" 105 in this menu and performs the clicking operation. This operation corresponds to the command to create the final result record (Step S2).

Upon receiving the command, the final result record report creator 45 reads the selected analysis data files as well as the audit trail report file and the analysis report file related to those analysis data files from the database 14 (Step S3). The correspondence relationship of the analysis data files with the audit trail report file and the analysis report file can be established by using the data ID which identifies each analysis data file as described earlier. As another possible method, when an audit trail report file or analysis report file is registered in the database 14, link information which shows the relationship with the analysis data files may be created and registered in the database 14 so that the audit trail report file and analysis report file concerned can be located by referring to this link information.

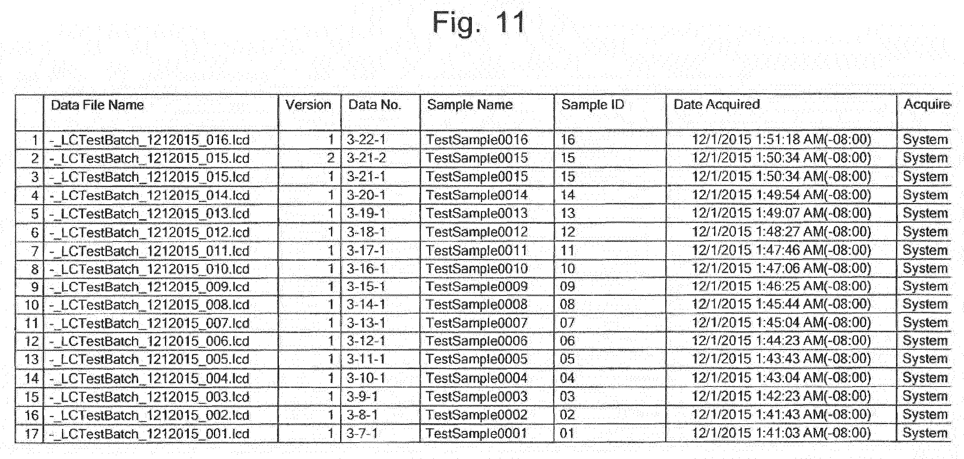

The data list creator 44 creates a data list showing the file name, sample name, sample ID and other items based on the data and information contained in the read analysis data files (Step S4). Fig. 11 shows one example of the data list (in part). It is preferable to allow users to arbitrarily set the items to be displayed in the data list.

According to the form of the final result record report specified in Step S1, the final result record report creator 45 creates an image of the final result record report in which the necessary contents are arranged, including the list of analysis results and re-analysis results prepared in Step S4, content of the audit trail report (list of the log information in time-series order) as shown in Fig. 10, content of the analysis report as shown in Fig. 9, as well as the analysis condition, data-analysis condition, calibration curve and other pieces of information contained in the analysis data files. The image of the final result record report is converted into a PDF file and registered in the database 14 (Step S5).

In this manner, a final result record report which holds an organized set of information and data related to a plurality of analysis data files selected by an operator can be created, and a PDF file of the report can be registered in the database 14.

On the other hand, in the case of using the "batch data set" setting in the file selection setting area 101 as shown in Fig. 5, a plurality of analysis data files to be included in one lot are previously specified and registered in a batch file. The batch file does not contain any substantial data (e.g. analysis results); it is a file which records the properties or similar information concerning a plurality of analysis data files.

The operator sets the conditions to narrow down the data sets in one or more narrowing items in the file selection setting area 101. Then, the batch files which satisfy the narrowing conditions are displayed in a batch file list 106 displayed in the file selection setting area 101. The operator indicates the target batch file among those batch files by a clicking operation with the mouse on the screen. In Fig. 5, the batch file indicated by reference sign 108 is selected. The selected file is highlighted.

Upon selection of a batch file, the analysis data files registered for the batch file are displayed in the data file list 102. The operator presses the right button of the mouse on this list, whereby an operation menu 108 as shown in Fig. 5 is displayed. The operator selects the "Create report set" 109 in this menu and performs the clicking operation. This operation corresponds to the command to create the final result record for all analysis data files specified in the form of a batch file. In this manner, the operator can collectively select a plurality of analysis data files to create the final result record report related to those analysis data files and register the report in the database 14.

When one final result record report corresponding to a plurality of analysis data files is created and stored in the database 14 in the previously described manner, the final result record report creator 45 creates link information showing the correspondence relationship between the final result record report and the original analysis data files. This link information is also registered in the database 14.

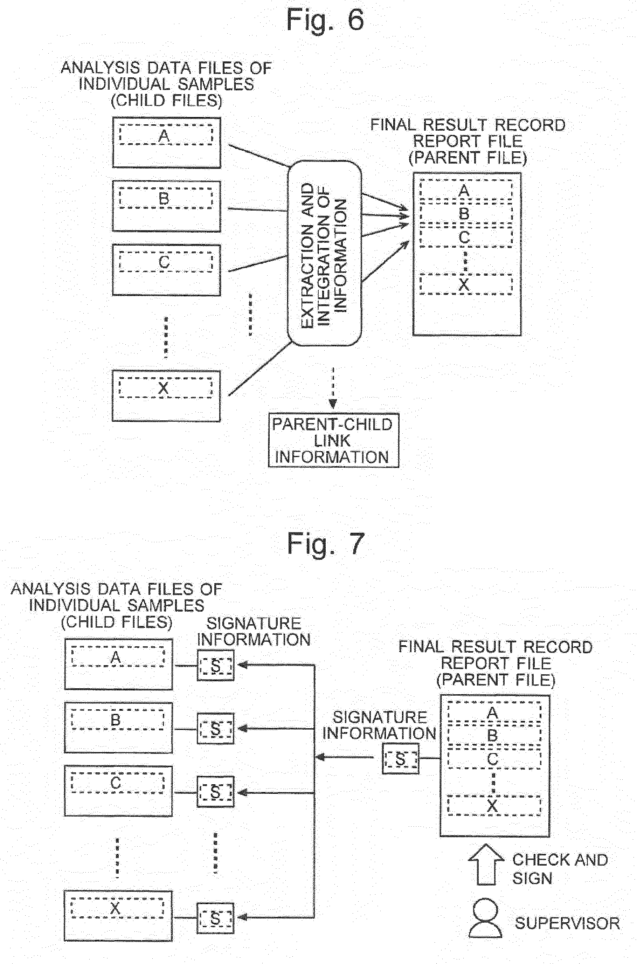

Fig. 6 is a conceptual diagram showing the relationship of files in the case of handling a plurality of analysis data files as one lot. As shown in Fig. 6, the information contained in one final result record report file obtained through the previously described process is composed of pieces of information A, B, ... and X which are parts of the analysis results and data-analyzing process results stored in the analysis data files as well as a graph, table or other forms of information based on those results. Accordingly, parent-child file link information which shows the relationship between a parent file and child files is created, with the final result record report file considered as a parent file and each analysis data file as a child file. In the case of using the "narrow down" setting in the file selection setting area 101, the analysis data files selected on the data manager window 100 correspond to the child files. In the case of using the "batch data set" setting in file selection setting area 101, the analysis data files included in the previously set batch file correspond to the child files.

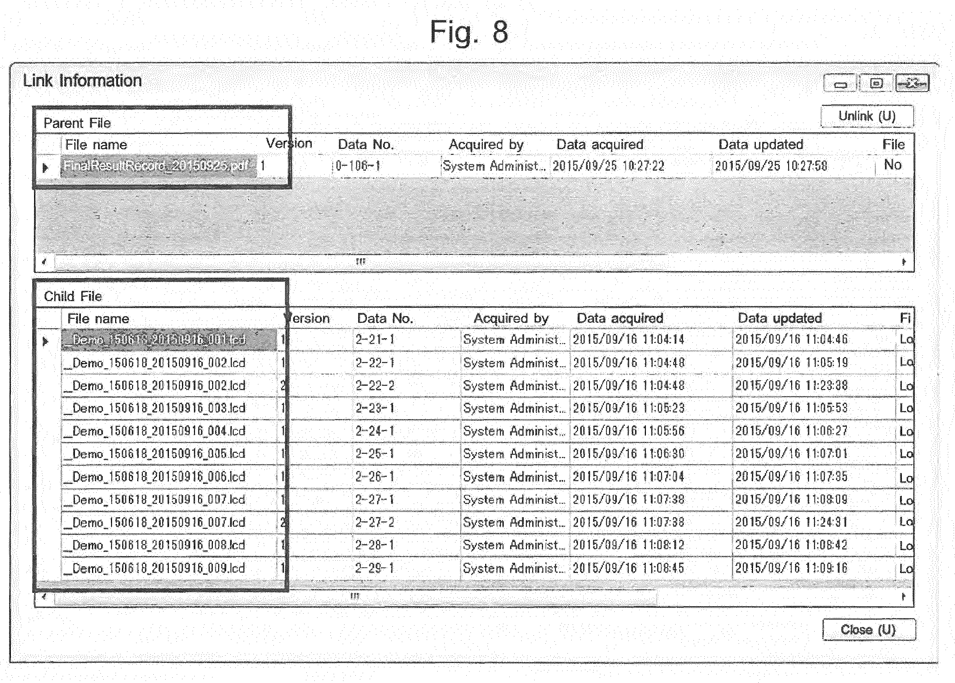

With the parent-child file link information prepared in this manner, a plurality of analysis data files corresponding to a final result record report file can be quickly located when the latter file is specified. The parent-child file link information can be visually checked as needed on the screen of the display unit 6 of the client terminal 4, for example, in a manner as shown in Fig. 8.

### [Execution of Signature of Approval, etc.]

The final result record report created in the previously described manner corresponds to the integrated file in the present invention. The report contains information for judging whether or not the data and data-analyzing process results in the analysis data files included in one lot are valid. A supervisor having a high level of administrative authority checks this final result record report to evaluate the validity of the analysis of the entire lot, and gives his/her signature. The procedure and processing operation for the signature are hereinafter described with reference to Fig. 7.

The supervisor performs a predetermined operation with the operation unit 5 of the client terminal 4, whereupon the signature-receiving processor 46 begins to operate and displays a final result record report file selection window on the display unit 6. For example, similar to the data manager window 100 shown in Fig. 4, the final result record report file selection window may preferably be configured to allow users to set appropriate narrowing conditions so as to extract only such files that satisfy the narrowing conditions among the final result record report files registered in the database 14, and display the extracted files in the form of a list. The supervisor selects a final result record report file to be checked from the list displayed on the display unit 6. The link information showing the relationship between the parent and child files as shown in Fig. 8 may also be displayed to help the supervisor select the correct final result record report file.

The signature-receiving processor 46 reads the selected final result record report file from the database 14 and displays, on the display unit 6, the final result record report based on the data contained in the file. For example, a chromatogram and a peak table based on the analysis result for each sample as well as the sample information, audit trail and other related information can be viewed on this display unit 6. The supervisor evaluates the validity of the analysis result for each sample and other contents in the displayed final result record report. Then, the supervisor performs a predetermined operation with the operation unit 5 to select a reason for the signature indicative of the approval, rejection or other evaluations of the content of the report, and performs the signing operation.

Specifically, for example, the supervisor selects one of the options of the reason prepared in a dropdown list and performs the clicking operation on the "Sign" button, whereupon a confirmation window pops up, allowing the supervisor to complete the signature by performing the clicking operation on the "OK" button. Upon detecting the predetermined operation mentioned earlier, the signature-receiving processor 46 recognizes that the signing has been carried out.

When the signature has been executed, the signature-executing processor 47 obtains signature information, including the signer's name, date and time of the signature as well as reason for the signature, and records the signature information, associating this information with the currently displayed final result record report file, i.e. the parent file. The signer's name is the name registered for the user ID with which the supervisor has logged in. The date and time of the signature is the information of the date and time at which the signing was carried out. The reason for the signature is the reason selected by the supervisor. The recording of the signature information can be performed, for example, by adding the signature information to the final result record report file and newly registering this file in the database 14 as a signed final result record report file. Another possibility is to write the signature information in a signature information file associated with the final result record report file, and register that file in the database 14.

Using the parent-child file link information, the signature-executing processor 47 locates a plurality of analysis data files corresponding to the final result record report file, i.e. the analysis data files from which the information contained the report file was extracted (or analysis report files or final result record report files which respectively correspond to the individual analysis data files), and records the same signature information as used for the final result record report file, associating this information with each of those analysis data files. The recording of the signature information can be achieved in the same manner as in the case of the final result record report file. Thus, the same signature information will be assuredly recorded in the final result record report file, which is the parent file, and in the analysis data files, which are the child files.

Additionally, a file containing signature information for a plurality of lots may be created, for example, in PDF format and registered in the database 14. This facilitates the task of checking the signature which indicates, for example, whether or not the final result record report for a certain lot has been approved.

Thus, with the analysis information management system according to the present embodiment, it is unnecessary to check the content of each of the analysis data files managed in lot units and sign each individual file. The signer can give his/her signature to the files after collectively checking and evaluating their contents.

It should be noted that the previous embodiment is a mere example of the present invention, and any modification, addition or change appropriately made within the spirit of the present invention will evidently fall within the scope of claims of the present application.

### REFERENCE SIGNS LIST

- 1: Database Server
- 11: Analysis Data Collector
- 12: Log Information Collector
- 13: Database Manager
- 14: Database
- 2: Analytical Instrument
- 3: Communication Network
- 4: Client Terminal
- 41: Data Analysis Processor
- 42: Analysis Report Creator
- 43: Audit Trail Report Creator
- 44: Data List Creator
- 45: Final Result Record Report Creator
- 46: Signature-Receiving Processor
- 47: Signature-Executing Processor
- 5: Operation Unit
- 6: Display Unit
- 7: Printing Unit

## Claims

1. An analysis information management system including a communication network, an analytical instrument connected to the communication network, a database server connected to the communication network, as well as a client terminal connected to the communication network and provided with an operation unit and a display unit, the analysis information management system configured to register, in a database constructed on the database server, a data file containing analysis data acquired by performing an analysis on a sample with the analytical instrument and/or data-analyzing process result acquired by performing a data-analyzing process based on the analysis data using the client terminal, and to manage the registered data file, wherein:
the client terminal includes:
a) an integrated file creator for extracting at least a portion of a plurality of pieces of information respectively contained in a plurality of data files registered in the database or a plurality of pieces of information respectively associated with the data files, for compiling the extracted pieces of information into one integrated file, and for storing the integrated file in the database;
b) a signature-receiving processor for reading the integrated file from the database and displaying the pieces of information contained in the integrated file on the display unit, and for receiving a predetermined operation with the operation unit by a user viewing the displayed information, as a command to execute a signature for the integrated file; and
c) a signature-executing processor for performing a recording of the signature for the integrated file and for performing a recording of the signature for each of the data files from which the integrated file is created, when the command to execute the signature is received by the signature-receiving processor.

2. The analysis information management system according to claim 1, wherein:
The integrated file creator creates link information showing a correspondence relationship between the integrated file and the data files from which the integrated file is created, and stores the link information in the database.

3. The analysis information management system according to claim 2, wherein:
the signature-receiving processor prepares a list of information for identifying the data files corresponding to the integrated file or information related to the data files, with reference to the link information, and displays the list on the display unit.
